# EUROPÄISCHE PATENTSCHRIFT

(11) **EP 0 457 222 B1**
(45) Veröffentlichungstag und Bekanntmachung des Hinweises auf die Patenterteilung: **30.11.1994**
(21) Anmeldenummer: 91107657.8
(22) Anmeldetag: 11.05.1991
(51) Int. Cl.: A61F 2/36

(54) **Femurprothese**
Hip prosthesis
Prothèse de la hanche

(30) Priorität: 18.05.1990 CH 1705/90
(43) Veröffentlichungstag der Anmeldung: 21.11.1991
(73) Patentinhaber: Intraplant AG, CH-6330 Cham (CH)
(72) Erfinder: Imhof, Martin, CH-6343 Rotkreuz (CH)
(74) Vertreter: Patentanwälte Schaad, Balass, Menzl & Partner AG

(56) Entgegenhaltungen:
- EP-A- 0 201 407
- EP-A- 0 339 530
- DE-U- 8 901 018
- FR-A- 2 574 283
- FR-A- 2 606 628

## Beschreibung

Die vorliegende Erfindung betrifft eine Femurprothese gemäss dem Oberbegriff des Patentanspruches 1, wie er aus der FR-A-2 606 628 bekannt ist.

Für ein gutes Resultat sind bei solchen Prothesen der CCD-Winkel (Centrum-Collum-Diaphysen-Winkel) und der Antetorsions-Winkel zwischen der Längsachse des Schaftblattes und der Achse des zur Aufnahme der Gelenkkugel bestimmten Aussenkonus den anatomischen Gegebenheiten des der Implantation der Prothesen bedürfenden Patienten anzupassen. Eine der Folgen dieser Erfordernisse besteht darin, dass bei den bisherigen, mit Ausnahme der Gelenkkugel einstückigen Femurprothesen dem Chirurgen eine ganze Anzahl solcher Prothesen mit unterschiedlichen CCD-Winkeln und unterschiedlichen Antetorsions-Winkeln zur Verfügung zu stehen hatte, damit für jeden spezifischen Fall die für eine richtige Verteilung der Kräfte an der implantierten Prothese am ehesten passende ausgelesen werden konnte. Dies bedeutet jedoch für den Hersteller einen nicht zu unterschätzenden Aufwand, da das Bestreben, eine möglichst genaue Anpassung an die im gegebenen Fall vorhandenen anatomischen Gegebenheiten zu ermöglichen, dazu führte, daß die dem Chirurgen zur Verfügung zu haltende Auswahl umso reichhaltiger zu sein hatte, auch wenn schliesslich nur eine Prothese aus dieser Auswahl zur Implantation gelangte.

Bei diesem Stand der Technik ist es eine Aufgabe der Erfindung, eine Prothese der eingangs genannten Art derart zu verbessern, dass sowohl der CCD-Winkel als auch der Antetorsions-Winkel in Grenzen verstellbar und feststellbar ist.

Diese Aufgabe ist bei der vorgeschlagenen Femurprothese dadurch gelöst worden, dass sie die im Kennzeichen des Patentanspruches 1 umschriebenen Merkmale aufweist.

Merkmale bevorzugter Ausführungsformen sind in den abhängigen Ansprüchen umschrieben.

Ausführungsbeispiele der vorgeschlagenen Femurprothese sind nachstehend anhand der Zeichnungen näher beschrieben.
Es zeigt:
- Fig. 1: eine AP-Ansicht, teilweise im Schnitt, einer Femurprothese mit aufgesetzter Gelenkkugel;
- Fig. 2: eine teilweise Draufsicht auf die Gelenkkugel der Prothese in Richtung des Pfeiles II der Fig. 1;
- Fig. 3: in grösserem Massstab ein Detail der Fig. 1;
- Fig. 4: eine Ansicht der aus Fig. 3 ersichtlichen Hülse aus Richtung des Pfeiles IV der Fig. 3;
- Fig. 5: eine Frontansicht, teilweise im Schnitt, einer anderen Ausführungsform einer Femurprothese mit aufgesetzter Gelenkkugel;
- Fig. 6: eine teilweise Draufsicht auf die Gelenkkugel der Prothese in Richtung des Pfeiles VI der Fig. 5;
- Fig. 7: in grösserem Massstab ein Detail der Fig. 5; und
- Fig. 8: eine Ansicht der aus Fig. 7 ersichtlichen Hülse aus Richtung des Pfeiles VIII der Fig. 7.

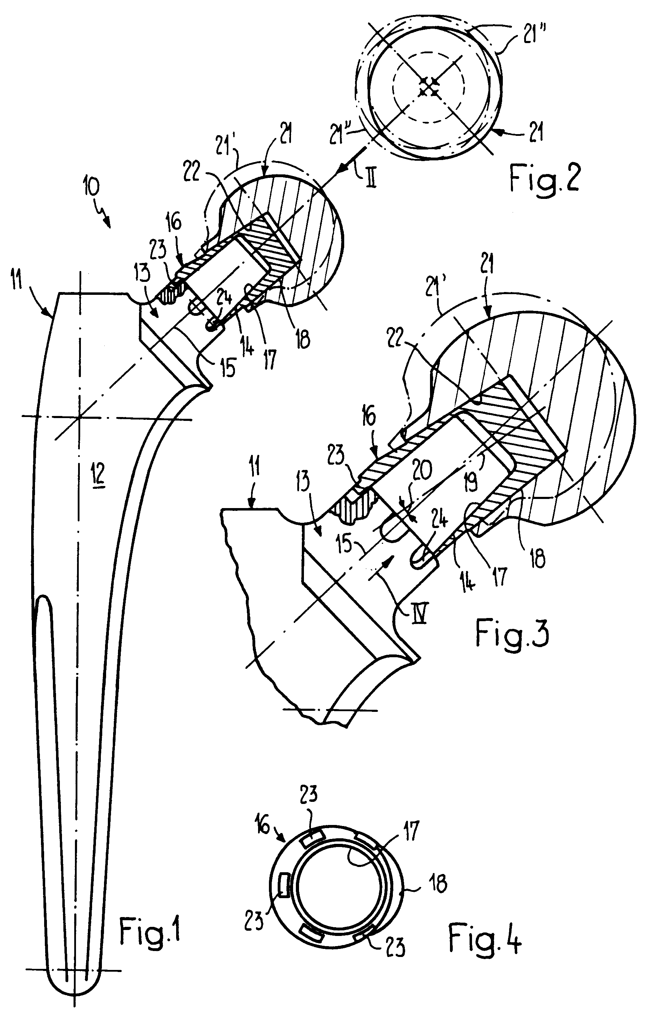

Es sei zunächst auf die Fig. 1 und 3 Bezug genommen. Die dargestellte Femurprothese 10 besitzt einen Schaft 11 mit einem Schaftblatt 12 und einem Hals 13. Am Hals 13 ist ein Aussenkonus 14 ausgebildet, dessen Achse mit 15 bezeichnet ist. Auf diesem Aussenkonus 14 sitzt eine Hülse 16, die einen gegengleich zum Aussenkonus 14 geformten Innenkonus 17 aufweist. Die Mantelfläche der Hülse ist als Aussenkonus 18 ausgebildet, dessen Achse in Fig. 3 mit 19 bezeichnet ist.

Aus den Fig. 1 und 3 ist deutlich zu erkennen, dass der Aussenkonus 18 nicht koaxial zum Innenkonus 17 und mithin zum Aussenkonus 14 ist. Vielmehr schneiden sich die Achsen 15 und 19 auf der Höhe der Mantelfläche der beiden Konen und schliessen zwischen sich einen kleinen, spitzen Winkel 20 in der Grössenordnung zwischen 5 und 10° ein, beispielsweise 7,5°.

Auf dem Aussenkonus 18 sitzt eine Gelenkkugel 21, in der ein zum Aussenkonus 18 gegengleicher Innenkonus 22 ausgebildet ist.

Am halsnahen Rand der Hülse 16 sind in regelmässigen Umfangsabständen Zähne 23 (vergleiche Fig. 4) ausgebildet. Andererseits sind anschliessend an den Aussenkonus 14 am Hals 13 ebenfalls in regelmässigen Umfangsabständen angeordnete Ausnehmungen 24 vorhanden, deren Breite jener der Zähne 23 entspricht. Bei aufgesetzter Hülse 16 greifen die Zähne 23 in die Ausnehmungen 24 und bilden somit eine Sicherung gegen eine Verdrehung der aufgesetzten Hülse 16.

Durch Abziehen der Hülse 16 vom Aussenkonus 14 und erneutem Aufsetzen derselben in einer anderen relativen Verdrehungslage gelingt es also, mit ein und demselben Schaft 11 Femurprothesen mit unterschiedlichen CCD-Winkeln und/oder unterschiedlichen Antetorsions-Winkeln auf einfachste Weise bereitzustellen.

Mit der strichpunktierten Linie 21' in Fig. 1 und 3 ist die Lage der Gelenkkugel 21 angedeutet, die diese bei um 180° verdreht aufgesetzter Hülse 16 einnimmt. Mit den strichpunktierten Linien 21'' in Fig. 2 ist dagegen der Umriss der Gelenkkugel 21 bei drei weiteren, unterschiedlichen Verdrehungslagen der aufgesetzten Hülse 16 angedeutet.

Zweckmässig werden die Offnungswinkel der Aussenkonen 14 und 18 gleich gross gewählt, um allenfalls die Gelenkkugel 21 auch direkt auf den Aussenkonus 14 aufsetzen zu können. Diese Öffnungswinkel sind so zu bemessen, dass sich ein fester Sitz auf den zugeordneten Innenkonen ergibt, also beispielsweise zwischen 5 und 6°.

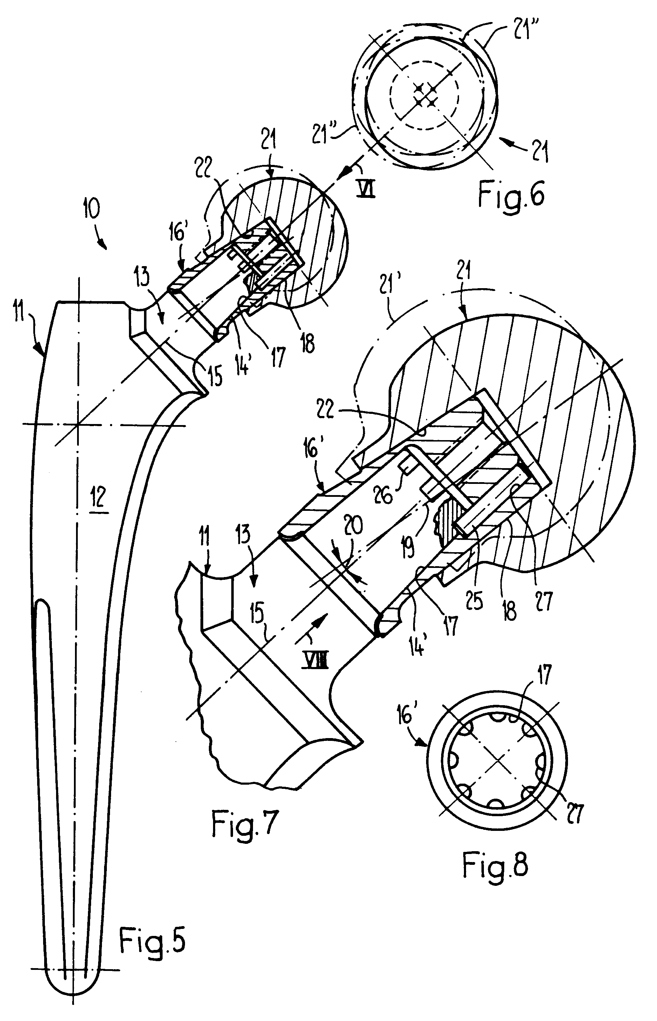

Die in den Figuren 5 bis 8 dargestellte Ausführungsform einer Femurprothese unterscheidet sich von derjenigen nach den Figuren 1 bis 4 durch die Mittel der Verdrehsicherung, die im Unterschied zu der vorstehend beschriebenen Ausführungsform nicht am schaftnanen Rand, sondern am bodenseitigen Ende der Hülse 16' gemäss der nachfolgenden Beschreibung angeordnet sind.

Am äusseren Ende des Aussenkonus 14' sind umfangsseitig Ausnehmungen 26 angeordnet, in die mindestens ein, vorzugsweise jedoch zwei Stifte 25 eingreifen, die im Boden der Hülse 16' verankert sind. Zur Verankerung sind in der Hülse 16' weitere Ausnehmungen und/oder Bohrungen 27 angeordnet, die sich jeweils in einer die Achse 19 der Hülse 16' schneidenden Ebene und parallel zur Achse 15 des Innenkonus 17 erstrecken.

Die als Verdrehsicherung dienende Rastanordnung 23,24; 25, 26 kann an irgendeiner geeigneten Stelle des Halses 13 angeordnet werden und nicht nur -wie gezeigt- am schaftnahen Ende der Hülse 16 bzw. am äusseren Ende des Aussenkonus 14' des Halses 13.

Statt die Hülse 16,16', wie in den Figuren dargestellt und vorstehend beschrieben, mittels ineinandergreifender Verzahnungen 23,24 bzw. Rastelemente 25,26 gegen Verdrehung zu sichern, ist es auch möglich, diese Verdrehsicherung durch Reibschluss zwischen Hülse 16,16' und Hals 13 zu bewirken, was durch entsprechende Ausbildung des Aussenkonus 14 und des Innenkonus 17 erreicht werden kann.

## Patentansprüche

1. Femurprothese mit einem Schaft (11), der einen Hals (13) mit einem Aussenkonus (14, 14') aufweist, wobei auf den Hals (13) wegnehmbar eine Hülse (16, 16') aufgesetzt ist, die einen gegengleich zum Aussenkonus (14, 14') des Halses (13) ausgebildeten Innenkonus (17) sowie einen zur Aufnahme einer Gelenkkugel (21) bestimmten, auf der Mantelfläche der Hülse (16, 16') ausgebildeten Aussenkonus (18) aufweist, dadurch gekennzeichnet, daß die Hülse (16, 16') in ihren jeweiligen Lagen gegen Verdrehung gesichert ist, und daß die Achsen (19, 15) des Aussenkonus (18) und des Innenkonus (17) der Hülse (16, 16') zwischen sich einen Winkel (20) einschliessen.

2. Femurprothese nach Patentanspruch 1, dadurch gekennzeichnet, dass die Achsen (19,15) des erstgenannten Aussenkonus (18) und des Innenkonus (17) sich im Bereich der Mantelfläche beider Konen schneiden.

3. Femurprothese nach Patentanspruch 1 oder 2, dadurch gekennzeichnet, dass der Öffnungswinkel des erstgenannten Aussenkonus (18) jenem des Innenkonus (17) entspricht.

4. Femurprothese nach Patentanspruch 1, gekennzeichnet durch Mittel (23,24;25,26) zum Sichern der Hülse (16,16') gegen Verdrehung.

5. Femurprothese nach Anspruch 4, dadurch gekennzeichnet, dass die Mittel zur Verdrehsicherung eine Rastanordnung mit sowohl an der Hülse (16,16') wie auch am Schaft (11) vorgesehenen, miteinander zusammenzuwirken bestimmten Rastelementen (23,24; 25,26) aufweisen.

6. Femurprothese nach Patentanspruch 5, dadurch gekennzeichnet, dass die Rastanordnung eine am schaftnahen Rand der Hülse (16) ausgebildete und eine am Hals (13) des Schaftes (11) angeformte Verzahnung (23,24) aufweisen, die bei aufgesetzter Hülse (16) ineinandergreifen.

7. Femurprothese nach Patentanspruch 5, dadurch gekennzeichnet, dass die Verdrehsicherung im Boden der Hülse (16') und am äusseren Ende des Aussenkonus (14') angeordnete Rastelemente (25,26) aufweisen, die bei aufgesetzter Hülse (16') ineinandergreifen.

8. Femurprothese nach Patentanspruch 7, dadurch gekennzeichnet, dass die Rastelemente durch am äusseren Ende des Aussenkonus (14') umfangsseitig angeordnete Ausnehmungen (26) gebildet sind, in die mindestens ein in der Hülse (16') verankerter Stift (25) eingreift.

9. Femurprothese nach Patentanspruch 7, dadurch gekennzeichnet, dass die Hülse (16') innenseitig weitere Ausnehmungen (27) und/oder Bohrungen zum Verankern der Stifte (25) aufweist, die sich jeweils in einer die Achse (19) der Hülse (16') schneidenden Ebene und parallel zur Achse (15) des Innenkonus (17) erstrecken.

10. Femurprothese nach Patentanspruch 1, dadurch gekennzeichnet, dass der Winkel (20) zwischen der Achse (19) des erstgenannten Aussenkonus (18) und jener (15) des Innenkonus (17) zwischen 5 und 10° beträgt.

11. Femurprothese nach Patentanspruch 6 oder 7, dadurch gekennzeichnet, dass die Verzahnungen (23,24) oder die Rastelemente (25,26) derart ausgebildet sind, dass die Hülse (16,16') in einer beschränkten Anzahl, beispielsweise in sechs oder acht Verdrehungslagen inbezug auf den zweitgenannten Aussenkonus (14,14') aufsetzbar ist.

12. Femurprothese nach einem der Patentansprüche 1 - 11, dadurch gekennzeichnet, dass die Hülse (16,16') durch Reibschluss gegen Verdrehung gesichert ist.

## Claims

1. A femoral prosthesis with a shank (11) comprising a neck (13) with an outer cone (14, 14'), whereby there is removably fitted onto the neck (13) a sleeve (16, 16') comprising an inner cone (17) constructed to be diametrically opposed to the outer cone (14, 14') of the neck (13) and also an outer cone (18) intended to accommodate an articulating ball (21) and constructed on the outer surface of the sleeve (16, 16'), characterised in that the sleeve (16, 16') is secured against rotation in whichever positions it occupies and in that the axes (19, 15) of the outer cone (18) and of the inner cone (17) of the sleeve (16, 16') enclose between them an angle (20).

2. A femoral prosthesis according to patent claim 1, characterised in that the axes (19, 15) of the first-mentioned outer cone (18) and of the inner cone (17) intersect in the region of the outer surface of both cones.

3. A femoral prosthesis according to patent claim 1 or 2, characterised in that the opening angle of the first-mentioned outer cone (18) corresponds to that of the inner cone (17).

4. A femoral prosthesis according to patent claim 1, characterised by means (23, 24; 25, 26) for securing the sleeve (16, 16') against rotation.

5. A femoral prosthesis according to claim 4, characterised in that the means of locking against rotation comprise a catch arrangement with catch elements (23, 24; 25, 26) intended to co-operate with one another and provided both on the sleeve (16, 16') and also on the shank (11).

6. A femoral prosthesis according to patent claim 5, characterised in that the catch arrangement comprises toothed arrangements (23, 24) integrally formed on the neck (13) of the shank (11) and constructed on the edge of the sleeve (16) which is close to the shank, said teeth interengaging when the sleeve (16) is fitted.

7. A femoral prosthesis according to patent claim 5, characterised in that the rotational locking means have in the bottom of the sleeve (16') and at the outer end of the outer cone (14') catch elements (25, 26) which interengage when the sleeve (16') is fitted.

8. A femoral prosthesis according to patent claim 7, characterised in that the catch elements are formed by recesses (26) disposed on the periphery at the outer end of the outer cone (14') and engaged by at least one pin (25) anchored in the sleeve (16').

9. A femoral prosthesis according to patent claim 7, characterised in that the sleeve (16') has on the inside further recesses (27) and/or bores for anchoring the pins (25) which extend in each case in a plane intersecting the axis (19) of the sleeve (16') and extending parallel with the axis (15) of the inner cone (17).

10. A femoral prosthesis according to patent claim 1, characterised in that the angle (20) between the axis (19) of the first-mentioned outer cone (18) and that (15) of the inner cone (17) amounts to between 5 and 10°.

11. A femoral prosthesis according to patent claim 6 or 7, characterised in that the sets of teeth (23, 24) or the catch elements (25, 26) are so constructed that the sleeve (16, 16') can be fitted in a limited number, for example six or eight, of rotated positions in respect of the second-mentioned outer cone (14, 14').

12. A femoral prosthesis according to one of patent claims 1 to 11, characterised in that the sleeve (16, 16') is secured against rotation by a frictionally locking arrangement.

## Revendications

1. Prothèse de la hanche, comprenant une tige (11) avec un col (13) pourvu d'un cône extérieur (14, 14'), sur le col (13) étant emboîtée de manière amovible une douille (16, 16') munie d'un cône intérieur (17) conjugué avec le cône extérieur (14, 14') du col (13) et d'un cône extérieur (18) conformé sur la surface latérale de la douille (16, 16') et destiné à recevoir une rotule (21), **caractérisée en ce** que la douille (16, 16') est bloquée en rotation dans ses positions respectives et que les axes (19, 15) du cône extérieur (18) et du cône intérieur (17) de la douille (16, 16') renferment entre eux un angle (20).

2. Prothèse de la hanche selon la revendication 1, caractérisée en ce que les axes (19, 15) du premier cône extérieur (18) et du cône intérieur (17) se coupent dans la région de la surface latérale des deux cônes.

3. Prothèse de la hanche selon l'une des revendications 1 ou 2, caractérisée en ce que l'angle d'ouverture du premier cône extérieur (18) correspond à celui du cône intérieur (17).

4. Prothèse de la hanche selon la revendication 1, caractérisée en ce qu'elle comporte des moyens (23, 24; 25, 26) pour le blocage en rotation de la douille (16, 16').

5. Prothèse de la hanche selon la revendication 4, caractérisée en ce que les moyens pour le blocage en rotation comprennent un dispositif à crans avec des éléments d'arrêt (23, 24; 25, 26) prévus sur la douille (16, 16') aussi bien que sur la tige (11) et destinés à coopérer les uns avec les autres.

6. Prothèse de la hanche selon la revendication 5, caractérisée en ce que le dispositif à crans comprend une denture (23, 24) conformée à chaque fois sur le bord de la douille (16) à proximité de la tige et sur le col (13) de la tige (11), lesdites dentures s'engageant l'une dans l'autre lorsque la douille (16) est mise en place.

7. Prothèse de la hanche selon la revendication 5, caractérisée en ce que le blocage en rotation comprend des éléments d'arrêt (25, 26) qui sont disposés dans le fond de la douille (16') et à l'extrémité extérieure du cône extérieur (14') et s'engagent les uns dans les autres lorsque la douille (16') est mise en place.

8. Prothèse de la hanche selon la revendication 7, caractérisée en ce que les éléments d'arrêt sont constitués par des évidements (26) disposés du côté périphérique à l'extrémité extérieure du cône extérieur (14') et dans lesquels s'engage au moins une broche (25) ancrée dans la douille (16').

9. Prothèse de la hanche selon la revendication 7, caractérisée en ce que la douille (16') présente intérieurement des évidements (27) et/ou des alésages supplémentaires pour l'ancrage des broches (25) qui s'étendent respectivement dans un plan qui coupe l'axe (19) de la douille (16') et est orienté parallèlement à l'axe (15) du cône intérieur (17).

10. Prothèse de la hanche selon la revendication 1, caractérisée en ce que l'angle (20) entre l'axe (19) du premier cône extérieur (18) et l'axe (15) du cône intérieur (17) est compris entre 5 et 10°.

11. Prothèse de la hanche selon l'une des revendications 6 ou 7, caractérisée en ce que les dentures (23, 24) ou les éléments d'arrêt (25, 26) sont conformés de telle façon que la douille (16, 16') peut être emboîtée dans un nombre de positions de rotation limité, par exemple six ou huit, par rapport au second cône extérieur (14, 14').

12. Prothèse de la hanche selon l'une des revendications 1 à 11, caractérisée en ce que la douille (16, 16') est bloquée en rotation par un entraînement par friction.
